# EUROPÄISCHE PATENTANMELDUNG

(11) **EP 0 103 252 A2**
(43) Veröffentlichungstag der Anmeldung: **21.03.1984**
(21) Anmeldenummer: 83108750.7
(22) Anmeldetag: 06.09.1983
(51) Int. Cl.: A01N 39/00, C07C 93/02, C07D 295/08, C07D 213/20, C07C 43/225

(54) **Aryloxyalkylamine, Verfahren zu ihrer Herstellung und ihre Verwendung zur Bekämpfung unerwünschten Pflanzenwuchses**

(30) Priorität: 11.09.1982 DE 3233828
(71) Anmelder: BASF Aktiengesellschaft, 67063 Ludwigshafen (DE)
(72) Erfinder: Sauter, Hubert, Dr., D-6800 Mannheim 1 (DE); Schirmer, Ulrich, Dr., D-6900 Heidelberg (DE); Tuerk, Wolfgang, Dr., D-6700 Ludwigshafen (DE); Wuerzer, Bruno, Dr., D-6701 Otterstadt (DE)

(57) **Zusammenfassung**

Die Erfindung betrifft Aryloxyalkylamine der Formel 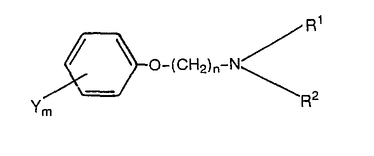 in der Y, m. n. R¹ und R² die in der Beschreibung genannten Bedeutungen haben. Verfahren zu ihrer Herstellung und ihre Verwendung zur Bekämpfung unerwünschten Pflanzenwuchses.

## Beschreibung

Die vorliegende Erfindung betrifft Aryloxyalkylamine, Verfahren zu ihrer Herstellung, Herbizide, die diese Verbindungen als Wirkstoffe enthalten, sowie ein Verfahren zur Bekämpfung unerwünschten Pflanzenwuchses mit diesen Verbindungen.

Es ist bekannt, daß w-(N,N-Dialkylamino)-alkyl-halogenphenylether herbizid wirksam sind (DE-OS 19 28 606). Außerdem sind Phenoxyalkylpiperidine bekannt, die als Antioxidantien Verwendung finden (C.A. 82, 16022 n (1975)).

Es wurde gefunden, daß Aryloxyalkylamine der Formel 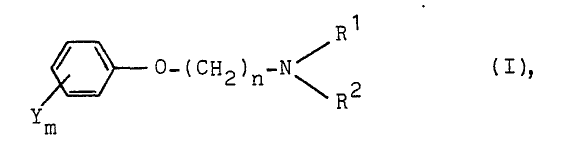 in der
Y Halogen, Cₗ-C₆-Alkyl, C₁-C₄-Halogenalkyl, Cₗ-C₄-Alkoxy, C₂-C₅-Alkanoyl, C₁-c₄-Alkylthio, C₁-C₄-Alkylsulfinyl, C₁-C₄-Alkylsulfonyl, Phenoxy,
Benzyloxy oder Cyano,
m 1, 2 oder 3,
n eine ganze Zahl von 5 bis 12 und
_{R}¹ und R² unabhängig voneinander Wasserstoff, gegebenenfalls durch C₁-C₄--Alkoxy, Hydroxy oder Cyano substituiertes C₁-C₄-Alkyl, C₂-C₄-Alkenyl, C₂--C₄-Alkinyl oder C₃-C₇-Cycloalkyl oder in der R¹ und R² zusammen eine gegebenenfalls durch Cₗ-C₄-Alkyl substituierte und/oder durch Sauerstoff oder Schwefel unterbrochene Alkylenkette mit 4 bis 7 Kohlenstoffkettengliedern oder in der R¹ und R² zusammen mit dem Stickstoff, dessen Substituenten sie sind, einen gegebenenfalls durch Halogen oder C₁-C₄-Alkyl substituierten Pyridiniumrest bedeuten
und in der anstelle des Restes 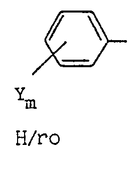 ein gegebenenfalls durch C₁-C₄-Alkyl, Formyl oder Hydroxy substituierter Naphthyl- oder 1,2,3,4-Tetrahydronaphthylrest stehen kann,
und Salze dieser Aryloxyalkylamine sowohl bei Vorauflauf- als auch bei Nachauflaufanwendung selektive herbizide Wirkstoffe sind.

Die in Formel I enthaltenen Substituenten können beispielsweise folgende Bedeutungen haben:
Y bedeutet Halogen, wie Fluor, Chlor, Brom, Iod, C₁-C₆-Alkyl, wie Methyl, Ethyl, t-Butyl, n-Hexyl, sec-Hexyl, C₁-C₄-Halogenalkyl, wie Trifluormethyl, Difluormethyl, 1,2,2-Trifluorethyl, C₁-C₄-Alkoxy, wie Methoxy, n-Propoxy, t-Butoxy, C₂-C₅-Alkanoyl, wie Acetyl, Propionyl, Phenoxy, Benzyloxy, Cyano,
m bedeutet 1, 2 oder 3, wobei die Substituenten Y gleich oder verschieden sein können, wenn m 2 oder 3 bedeutet,
n bedeutet eine ganze Zahl von 5 bis 12, vorzugsweise von 6 bis 10,
R¹ und R² bedeuten unabhängig voneinander Wasserstoff, gegebenenfalls durch C₁-C₄-Alkoxy, Hydroxy oder Cyano substituiertes C₁-C₄-Alkyl, wie Methyl, Ethyl, Isopropyl, n-Propyl, n-Butyl, s-Butyl, t-Butyl, n-Butyl, i-Butyl, s-Pentyl, 2-Methoxyethyl, 2-Cyanoethyl, 2-Hydroxyethyl, C₂-C₄--Alkenyl oder C₂-C₄-Alkinyl, wie Allyl, Propargyl, 1-Methyl-n-prop-2--inyl, C₃-C₇-Cycloalkyl, wie Cyclopropyl, Cyclopentyl, Cyclohexyl. Rⁱ und R² können auch zusammen eine gegebenenfalls durch C₁-C₄-Alkyl substituierte und/oder durch Sauerstoff oder Schwefel unterbrochene Alkylenkette mit 4 bis 7 Kohlenstoffkettengliedern bedeuten, wie Tetramethylen, Pentamethylen, Hexamethylen, 1,4-Dimethyl-tetramethylen, 3-0xa-pentamethylen, 3-Thiapentamethylen. Weiterhin können R¹ und R² zusammen mit dem Stickstoff, dessen Substituenten sie sind, einen gegebenenfalls durch Halogen, wie Chlor, oder C₁-C₄-Alkyl, wie Methyl, Ethyl, t-Butyl, substituierten Pyridiniumrest, bilden, beispielsweise 3-Chlor-pyridinium, 4-Methyl-pyridinium, 4-t-Butyl-pyridinium.

Anstelle des Restes 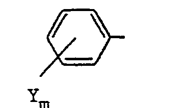 können die Verbindungen der Formel I auch einen gegebenenfalls durch ^{C}₁-C₄-Alkyl,

vorzugsweise Methyl, durch Formyl oder Hydroxy substituierten Naphthylrest, wie Naphth-1-yl, Naphth-2-yl, 1-Methyl-naphth-2-yl, 2-Hydroxy-1--naphthaldehyd, 2-Hydroxy-naphth-1-yl, oder einen gegebenenfalls durch C₁-C₄-Alkyl, vorzugsweise Methyl, durch Formyl oder Hydroxy substituierten 1,2,3,4'-Tetrahydro-naphthylrest, wie 1,2,3,4-Tetrahydro-naphth-8-ol, enthalten.

Aryloxyalkylamine der Formel 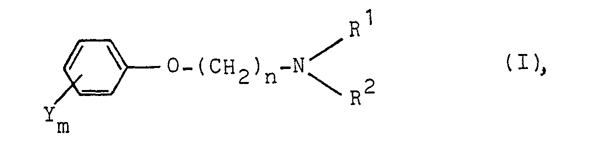 in der
Y Halogen, C₁-C₆-Alkyl, C₁-C₄-Halogenalkyl, C₁-C₄-Alkoxy, C₂₋C_{S}-Alkanoyl, C₁-C₄-Alkylthio, Cₗ-C₄-Alkylsulfinyl, C₁-C₄-Alkylsulfonyl, Phenoxy, Benzyloxy oder Cyano,
m 1, 2 oder 3,
n eine ganze Zahl von 5 bis 12,
_{R}¹ und _{R}² unabhängig voneinander Wasserstoff, gegebenenfalls durch C₁-C₄--Alkoxy, Hydroxy oder Cyano substituiertes C₁-C₄-Alkyl, C₂-C₄-Alkenyl, C₂-C₄-Alkinyl oder C₃-C₇-Cycloalkyl oder in der R¹ und R² zusammen eine gegebenenfalls durch C₁-C₄-Alkyl substituierte und/oder durch Sauerstoff oder Schwefel unterbrochene Alkylenkette mit 4 bis 7 Kohlenstoffkettengliedern oder in der R¹ und R² zusammen mit dem Stickstoff, dessen Substituenten sie sind, einen gegebenenfalls durch Halogen oder C_{I}-C₄-Alkyl substituierten Pyridiniumrest bedeuten
und in der anstelle des Restes 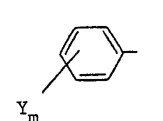 ein gegebenenfalls durch C₁-C₄-Alkyl,

Formyl oder Hydroxy substituierter Naphthyl- oder 1,2,3,4-Tetrahydro--naphthylrest stehen kann,
mit der Maßgabe, daß n nicht 6 bedeutet, wenn Y t-Butyl und R¹ und R² zusammen Pentamethylen bedeuten,
und Salze dieser Aryloxyalkylamine sind neu.

Bevorzugte Aryloxyalkylamine der Formel I sind solche, bei denen Y Halogen, wie Chlor oder Brom, m 1, n eine ganze Zahl von 6 bis 10 und R¹ und R² unabhängig voneinander C₁-C₄-Alkyl, beispielsweise Ethyl oder Isopropyl, bedeuten, sowie die Säureadditionssalze dieser Verbindungen Herbizid gut wirksame Salze der Aryloxyalkylamine der Formel I, wobei die Gruppe 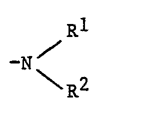 einen Pyridiniumrest bedeutet, sind solche, bei denen Y Halogen, wie Chlor, oder Cₗ-C₄-Alkyl, wie Methyl, m 2, n eine ganze Zahl von 6 bis 10 und R¹ und R² zusammen mit dem Stickstoff, dessen Substituenten sie sind, einen gegebenenfalls durch Halogen, wie Chlor oder Brom, oder C₁-C₄--Alkyl, wie Methyl, substituierten Pyridiniumrest bedeuten.

Die Aryloxyalkylamine der Formel I können in an sich bekannter Weise durch Umsetzung von Phenoxyalkylhalogeniden der Formel 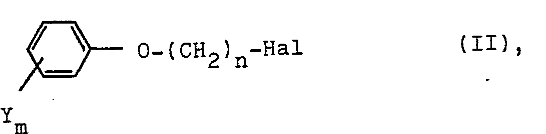 in der Y, m und n die obengenannten Bedeutungen haben und Hal für Halogen, insbesondere für Chlor oder Brom steht, mit Aminen der Formel 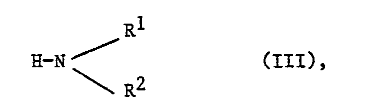 in der _{R}^{I}, R² und n die obengenannten Bedeutungen haben und Hal für Halogen steht, erhalten werden.

Die Umsetzung wird bei einer Temperatur im Bereich von 40 bis 150°C, vorzugsweise 60 bis 120^{o}C, durchgeführt.

Zweckmäßigerweise wird sie in Gegenwart eines inerten Lösungsmittels durchgeführt. Hierbei werden die Reaktanden in Gegenwart des Lösungsmittels erhitzt und das Endprodukt wird im Anschluß daran durch Destillation abgetrennt bzw. nach Bildung des Säureadditionssalzes abgetrennt.

Geeignete Lösungsmittel sind Alkohole, wie Methanol, Ethanol, Isopropanol, t-Butanol, aliphatische und aromatische Kohlenwasserstoffe, wie n-Hexan, Toluol, Xylole, Decalin, 1,2,3,4-Tetrahydronaphthalin, Amide, sowie Dimethylformamid, Dimethylacetamid, cyclische und acyclische Ether, wie Diethylether, Methyl-t-butylether, Dioxan, Tetrahydrofuran, sowie N-Methylpyrrolidon.

Die Menge an Lösungsmittel, bezogen auf Phenoxyalkylhalogenid der Formel II, beträgt 10 bis 80 Gew.%.

Das Verfahren kann auch so durchgeführt werden, daß man das substituierte Phenoxyalkylhalogenid mit einem Überschuß des jeweiligen Amins in einem Reaktionsgefäß erhitzt. Die Isolierung des Produktes aus dem Reaktionsgemisch erfolgt dann in Form der freien Base oder des Säureadditionssalzes des erhaltenen substituierten Phenoxyalkylamins.

Man erhält die Aryloxyalkylamine der Formel I auch durch Umsetzung eines Aminoalkylhalogenids der Formel 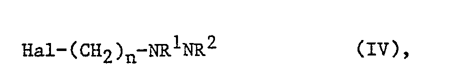 in der
R^{l}, _{R}² und n die obengenannten Bedeutungen haben und Hal für Halogen, insbesondere für Chlor oder Brom, steht, mit einem Alkalisalz eines Phenols der Formel 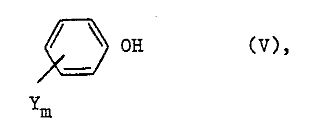 in der Y und m die obengenannten Bedeutungen haben.

Die Umsetzung wird in Gegenwart eines inerten Lösungsmittels bei einer Temperatur im Bereich von 50 bis 200°C, vorzugsweise 60 bis 140^{o}C, durchgeführt.

Geeignete Lösungsmittel sind Alkohole, wie Methanol, Ethanol, Isopropanol, acyclische und cyclische Ether, wie Methyl-t-butylether, Dioxan, Tetrahydrofuran, aromatische Kohlenwasserstoffe, wie Toluol, Xylole, Chlorbenzol, sowie Wasser. Die Menge an Lösungsmittel, bezogen auf Phenolat, beträgt 10 bis 80 Gew.%.

Die zur Synthese der Verbindungen der Formel I als Vorprodukte benötigten substituierten Phenole, Aminoalkylhalogenide, Phenoxyalkylhalogenide und Amine sind im Handel erhältlich; sie lassen sich nach literaturbekannten, üblichen Methoden synthetisieren.

Die Phenoxyalkylhalogenide der Formel II können nach der in J. Med. Chem. 14, 133 (1971) beschriebenen Methode hergestellt werden. Zur Synthese von beispielsweise 1-(4-Methylphenoxy)-10-brom-n-decan erhitzt man eine Lösung von 4-Methylphenol, Kaliumhydroxid und 1,10-Dibromdecan in Methanol über Nacht auf Rückflußtemperatur. Das Reaktionsgemisch wird dann abgekühlt und filtriert, worauf man das Filtrat zur Entfernung des Lösungsmittels unter vermindertem Druck einengt. Das dabei zurückbleibende rohe flüssige Material wird in Ether gelöst, und die Etherlösung wird dann mit verdünnter wäßriger Natriumhydroxidlösung und dann mit Wasser solange gewaschen, bis die Waschflüssigkeiten neutral sind.

Die dabei anfallende organische Schicht wird dann über einem geeigneten Trockenmittel (z.B. Na₂S0₄) getrocknet, filtriert und unter vermindertem Druck destilliert, wodurch man zu einem bei etwa 165⁰C unter einem Druck von 0,25 mbar siedenden Produkt gelangt, das als 1-(4-Methylphenoxy)-10--brom-n-decan identifiziert wird.

Zur Herstellung der Aminoalkylhalogenide der Formel IV läßt man ein Aminoalkanol mit einem Halogenierungsmittel, wie Thionylchlorid oder Phosphoroxybromid, in einem geeigneten Lösungsmittel reagieren und isoliert anschließend das gewünschte Aminoalkylhalogenid als Hydrohalogenidsalz (J. Med. Chem. 14, 133 (1971)).

Die Herstellung der Säureadditionssalze der Verbindungen der Formel I, bei denen die Gruppe -NR¹R² kein Pyridiniumrest ist, erfolgt ausgehend von einer Säure solcher Acidität, daß hierdurch der Aminorest der jeweiligen Verbindung reagiert. Hierzu geeignete Säuren sind Chlorwasserstoffsäure, Bromwasserstoffsäure, Salpetersäure, Schwefelsäure, Oxalsäure, Methansulfonsäure oder p-Toluolsulfonsäure. Die Herstellung von Säureadditionssalzen wird in an sich bekannter Weise durchgeführt.

Die Pyridiniumsalze der Formel I lassen sich durch Umsetzung der Aryloxyalkylamine der Formel II mit den entsprechenden substituierten Pyridinverbindungen herstellen. In Betracht kommen insbesondere Bromide, Chloride, Iodide.

Die folgenden Beispiele erläutern die Herstellung der Aryloxyalkylamine der Formel I.

### Beispiel 1

### 1-(4-Methylphenoxy)-10-(N,N-diethylamino):decan (Verbindung Nr. 1)

Ein Gemisch aus 36,6 g Diethylamin und 14,6 g 1-(4-.Methylphenoxy)-10--brom-n-decan werden in ölbad über Nacht unter Rückfluß erhitzt. Nach dem Abkühlen wird das Reaktionsgemisch mit Ether und 20 %iger Natriumhydroxidlösung aufgenommen. Dann extrahiert man das Gemisch mit Ether und trocknet die gesammelten organ. Phasen über Natriumsulfat. Nach dem Verdampfen des Lösungsmittels bleiben 14,1 g öl zurück, die an einer Säule mit Silikagel 60 (0,063 bis 0,2 mm) und einem Lösungsmittelgemisch aus Methylenchlorid/Methanol (9:1) chromatographiert wird. Man erhält 5 g 1-(4-Methylphenoxy)-10-(N,N-diethylamino)-decan des durch 1_{H-NMR-} und IR-Spektroskopie identifiziert wird:
1_{H-NMR} (100 MHz; CDCl₃; TMS) 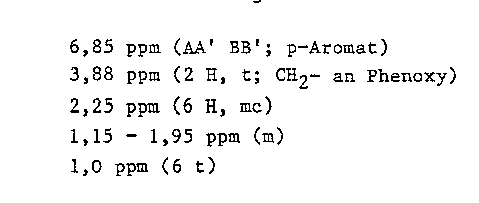
IR (CHC1₃; cm⁻¹) ₂97₀, 293₀, 286₀, 28₀₀, 1512, 1470, 1385, 1295, 1245, 1170, 800, 820

### Beispiel 2

### 1-(4-Chlorphenoxy)-10-piperidinyl-n-decan (Verbindung Nr. 2)

Ein Gemisch aus 24,7 g Piperidin und 10,1 g 4-Chlorphenoxy-n-decylbromid werden im ölbad über Nacht unter Rückfluß erhitzt. Nach dem Abkühlen wird das Reaktionsgemisch mit Ether und 20 %iger Natriumhydroxidlösung aufgenommen. Dann extrahiert man das Gemisch mit Ether und trocknet die gesammelten organischen Phasen über Natriumsulfat. Nach dem Verdampfen des Lösungsmittels erhält man 9,5 g öl, das durch ^{I}H-NMR- und IR-Spektroskopie als 1-(4-Chlorphenoxy)-10-piperidinyl-n-decan identifiziert wird:
NMR (100 MHz; CDC1₃; TMS)

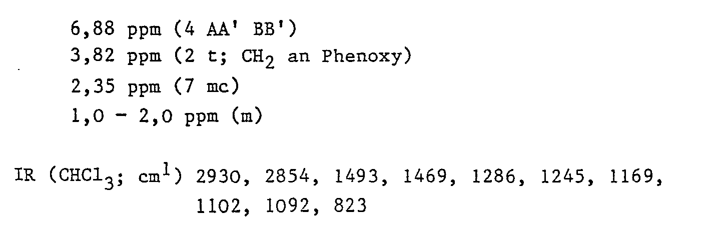

### Beispiel 3

### N-[6-(2-Methyl-4-chlor-phenoxy)-n-hexyl]-4-methyl-pyridiniumbromid (Verbindung Nr. 91)

Eine Lösung von 185,2 g 2-Methyl-4-chlorphenol, 57 g Kaliumhydroxid und 388,4 g 1,6-Dibrom-n-hexen in 600 ml Methanol wird über 48 Stunden auf Rückflußtemperatur erhitzt. Das Reaktionsgemisch wird dann abgekühlt, filtriert und unter vermindertem Druck eingeengt. Anschließend wird der Rückstand mit Wasser aufgenommen und mit Diethylether (500 ml) zweimal extrahiert. Die Etherphase wird nochmals mit 10 Xiger Natriumhydroxidlösung gewaschen und über Natriumsulfat getrocknet. Das Lösungsmittel wird destilliert, und der Rückstand wird unter vermindertem Druck fraktioniert destilliert, wobei ein bei 162 bis 163°C unter einem Druck von 0,-13 mbar siedendes Produkt erhalten wird.

27,5 g des so erhaltenen 6-(2-Methyl-4-chlorphenoxy)-1-brom-n-hexan und 8,4 g 4-Methylpyridin werden in äquimolaren Mengen in 200 ml Acetonitril auf 82°C erhitzt. Eine Dünnschichtkontrolle zeigt das Ende der Reaktion an. Nach Abdestillieren des Lösungsmittels wird der anfallende Kristallbrei mit Diethylether aufgerührt und aus Methanol umkristallisiert. Die isolierten Kristalle haben einen Festpunkt von 63 bis 65°C; Ausbeute 29,1 g.

Entsprechend können folgende Aryloxyalkylamine der Formel I erhalten werden. In den folgenden Tabellen bedeuten

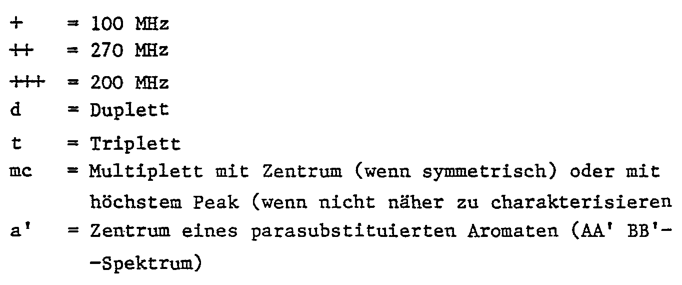

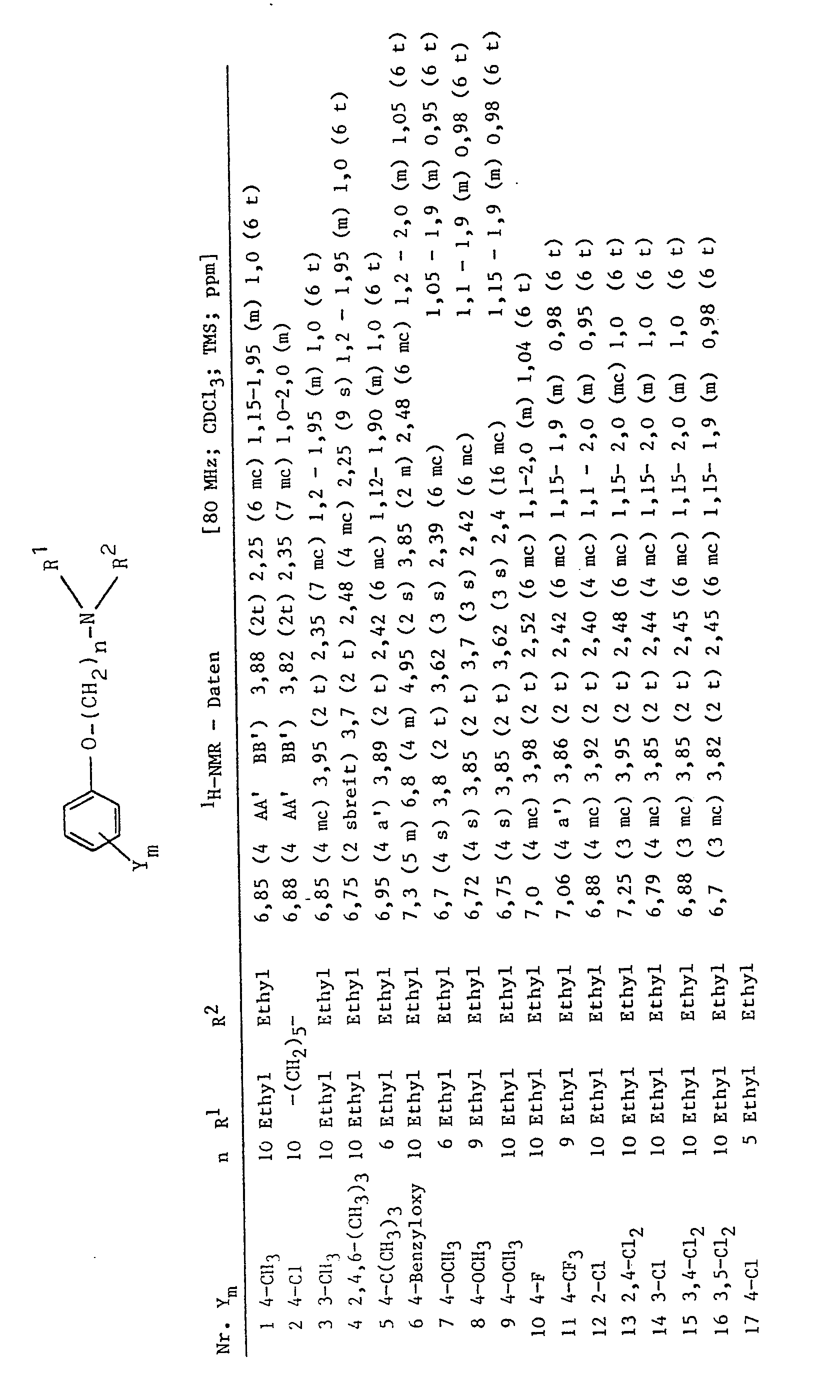

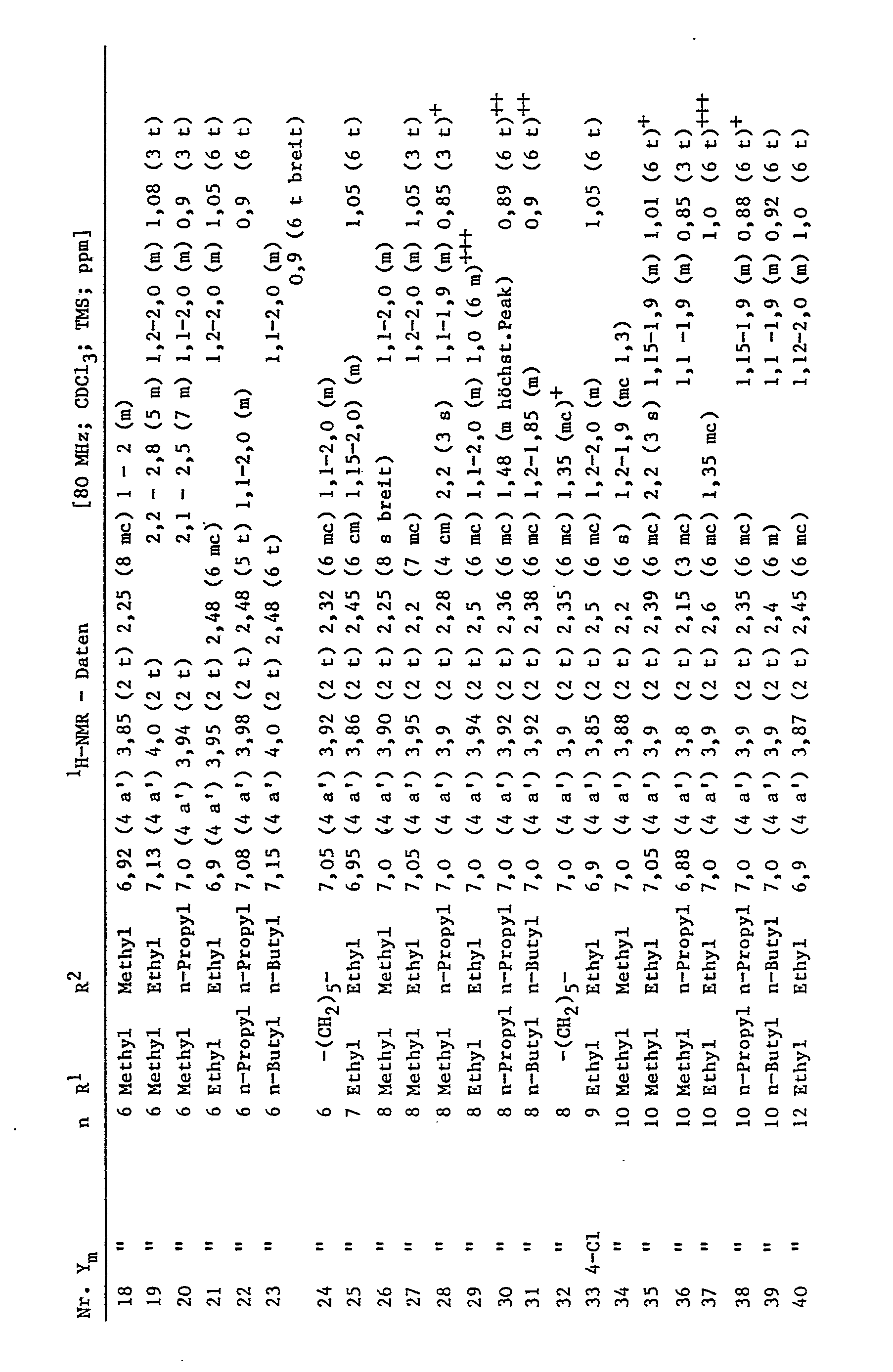

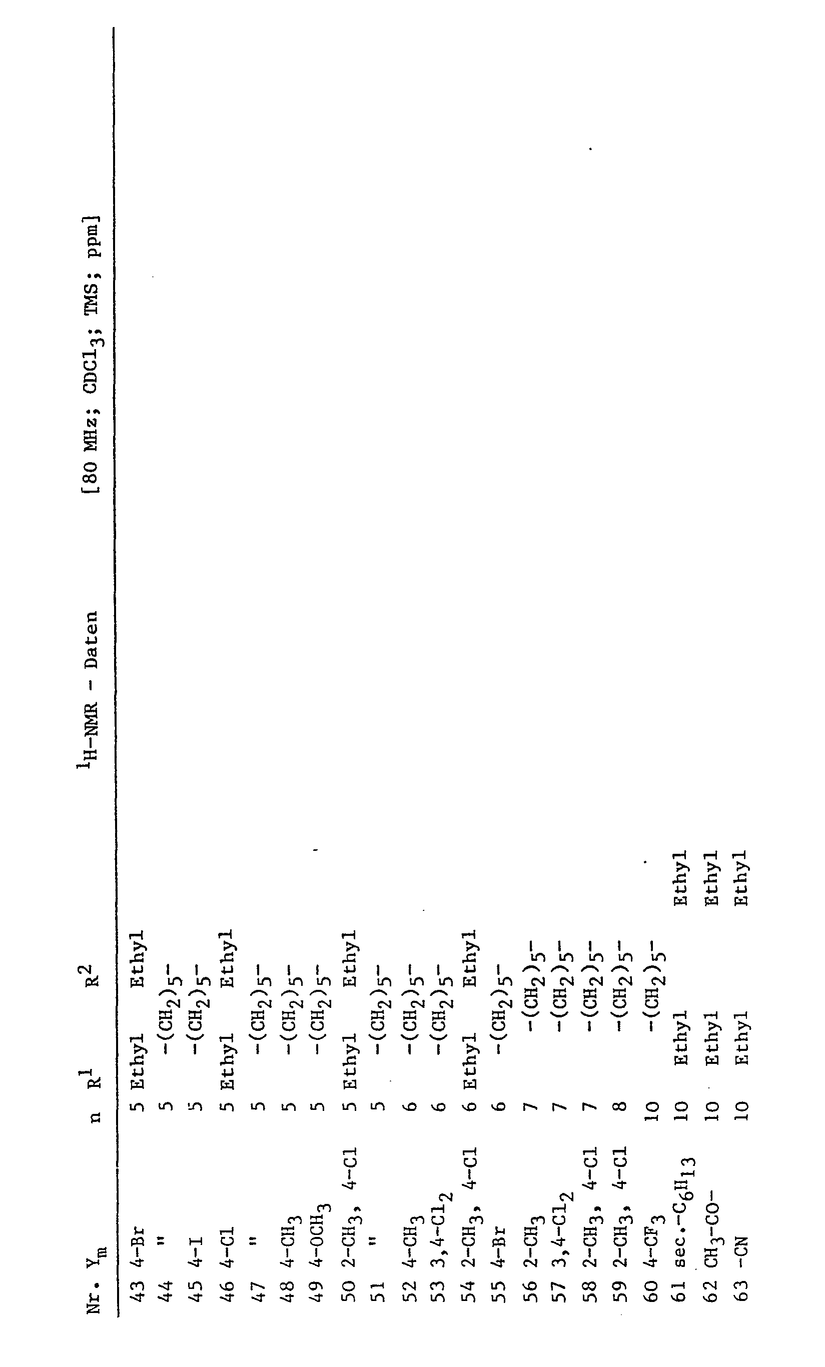

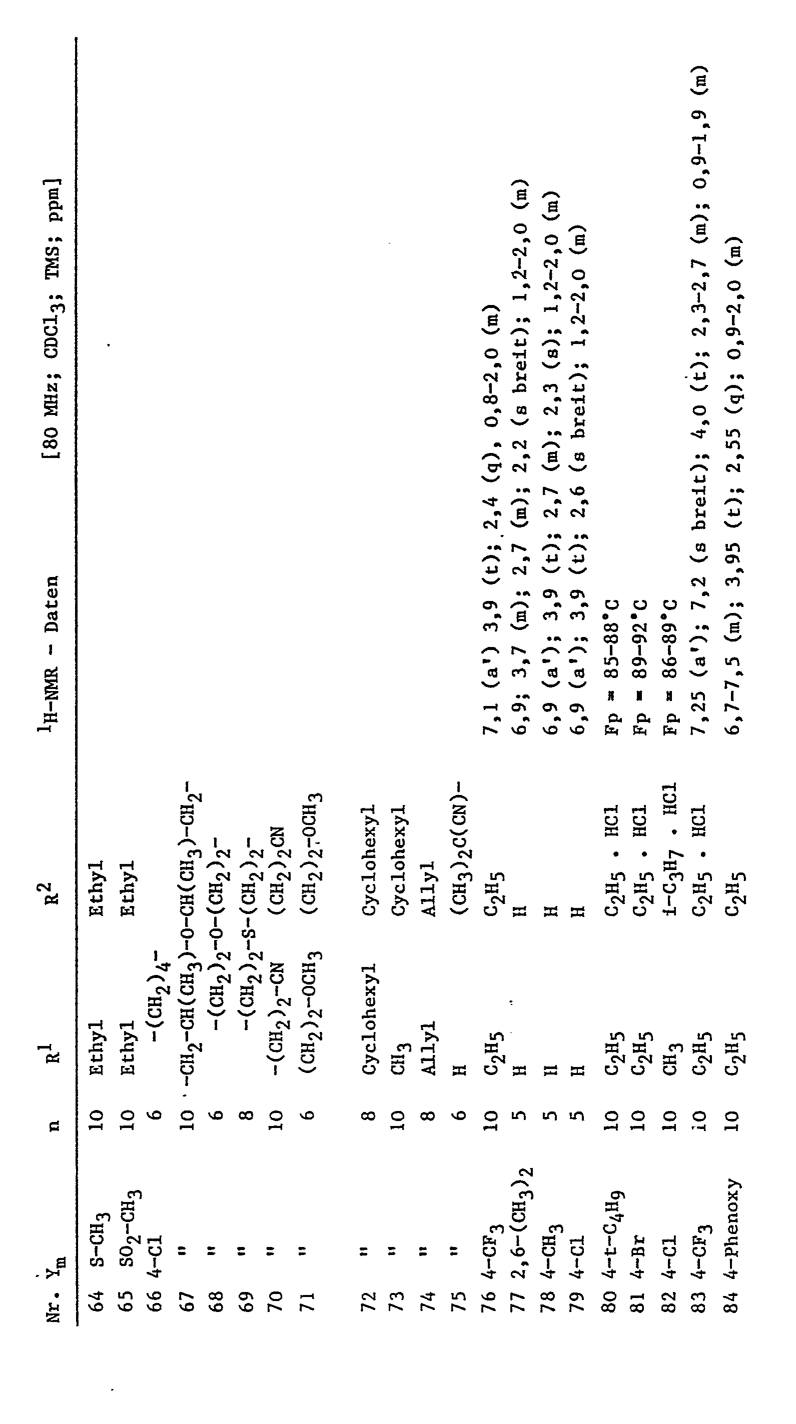

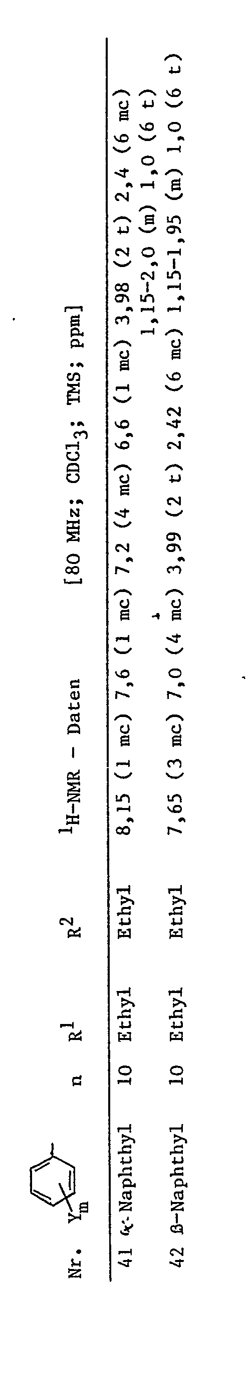

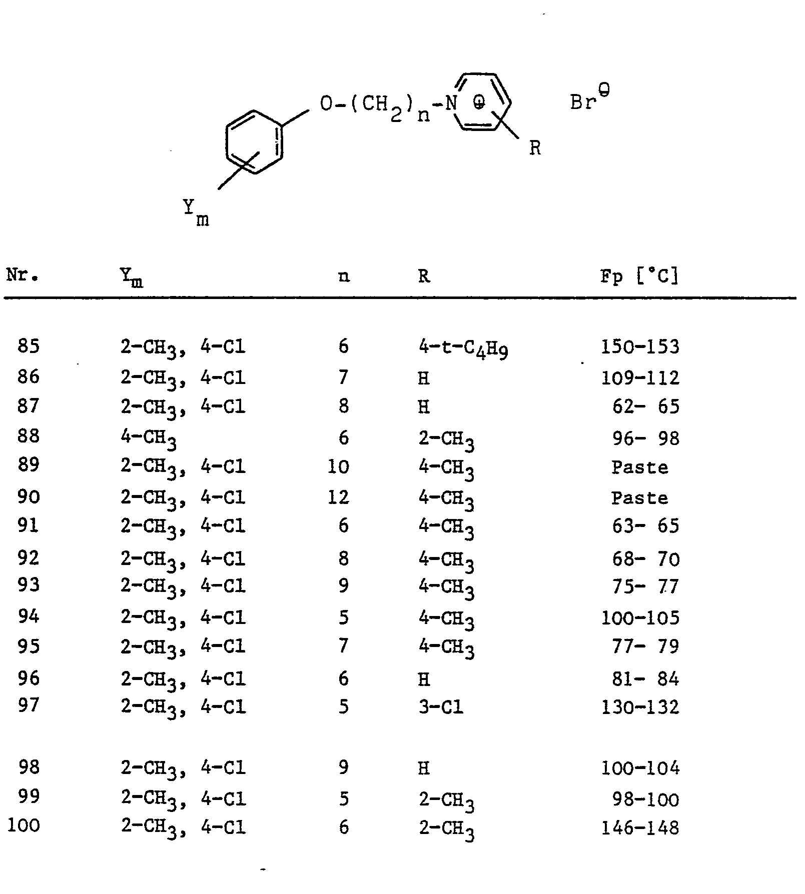

Die Verbindungen der Formel I oder deren Salze können beispielsweise in Form von direkt versprühbaren Lösungen, Pulvern, Suspensionen, auch hochprozentigen wäßrigen, öligen oder sonstigen Suspensionen oder Dispersionen, Emulsionen, öldispersionen, Pasten, Stäubemitteln, Streumitteln oder Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder öldispersionen kommen Mineralölfraktionen von mittlerem bis hohem Siedepunkt, wie Kerosin oder Dieselöl, ferner Kohlenteeröle sowie öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, z.B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, öldispersionen) durch Zusatz von Wasser bereitet werden. Zur Herstellung von Emulsionen, Pasten oder öldispersionen können die Substanzen als solche oder in einem öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenoläther, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate, können durch Bindung der Wirkstoffe an festen Trägerstoffen hergestellt werden. Feste Trägerstoffe sind Mineralerden wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten zwischen 0,1 und 95 Gewichtsprozent, vorzugsweise zwischen 0,5 und 90 Gewichtsprozent, Wirkstoff.

Beispiele für Formulierungen sind:
I. Man vermischt 90 Gewichtsteile der Verbindung Nr. 21 mit 10 Gewichtsteilen N-i_{M}eth^{y}ₗ-cL-pyrrolidon und erhält eine Lösung, die zur Anwendung in Form kleinster Tropfen geeignet ist.
II. 20 Gewichtsteile der Verbindung Nr. 33 werden in einer Mischung gelöst, die aus 80 Gewichtsteilen Xylol, 10 Gewichtsteilen des Anlagerungsproduktes von 8 bis 10 Mol Ethylenoxid an 1 Mol ölsäure-N--monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gewichtsprozent des Wirkstoffs enthält.
III. 20 Gewichtsteile der Verbindung Nr. 25 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteile des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.
IV. 20 Gewichtsteile der Verbindung Nr. 29 werden in einer Mischung gelöst, die aus 25 Gewichtsteilen Cyclohexanol, 65 Gewichtsteilen einer Mineralölfraktion vom Siedepunkt 210 bis 280⁰C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.
V. 20 Gewichtsteile der Verbindung Nr. 37 werden mit 3 Gewichtsteilen des Natriumsalzes der Diisobutylnaphthalin-ol-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gewichtsprozent des Wirkstoffs enthält.
VI. 3 Gewichtsteile der Verbindung Nr. 18 werden mit 97 Gewichtsteilen feinteiligem Kaolin vermischt. Man erhält auf diese Weise ein Stäubemittel, das 3 Gewichtsprozent des Wirkstoffs enthält.
VII. 30 Gewichtsteile der Verbindung Nr. 19 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.
VIII. 20 Teile der Verbindung Nr. 87 werden mit 2 Teilen Calciumsalz der Dodecylbenzolsulfonsäure, 8 Teilen Fettalkohol-polyglykolether, 2 Teilen Natriumsalz eines Phenol-Harnstoff-Formaldehyd-Kondensates und 68 Teilen eines paraffinischen Mineralöls innig vermischt. Man erhält eine stabile ölige Dispersion.

Die Applikation der Wirkstoffe bzw. der Mittel kann im Vorauflaufverfahren oder im Nachauflaufverfahren erfolgen. Sind die Wirkstoffe für die Kulturpflanzen weniger verträglich, so können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Jahreszeit, Bekämpfungsziel und Wachstumsstadium und Bodenart 0,1 bis 5 kg/ha und mehr, vorzugsweise 0,25 bis 3 kg/ha.

Die herbizide Wirkung von Verbindungen der Formel I wird durch Gewächshausversuche gezeigt:
Als Kulturgefäße dienen Plastikblumentöpfe mit 300 cm³ Inhalt und lehmigem Sand mit etwa 1,5 % Humus als Substrat. Die Samen der Testpflanzen werden nach Arten getrennt flach eingesät. Unmittelbar danach erfolgt bei Vorauflaufbehandlung das Aufbringen der Wirkstoffe auf die Erdoberfläche. Sie werden hierbei in Wasser als Verteilungsmittel suspendiert oder emulgiert und mittels fein verteilender Düsen gespritzt. Bei dieser Applikationsmethode beträgt die Aufwandmenge 2,0 oder 3,0 kg Wirkstoff/ha. Nach dem Aufbringen der Mittel werden die Gefäße leicht beregnet, um Keimung und Wachstum in Gang zu bringen. Danach deckt man die Gefäße mit durchsichtigen Plastikhauben ab, bis die Pflanzen angewachsen sind. Die Abdeckung bewirkt ein gleichmäßiges Keimen der Testpflanzen, sofern dies nicht durch die Wirkstoffe beeinträchtigt wird.

Zum Zwecke der Nachauflaufbehandlung zieht man die Testpflanzen je nach Wuchsform bis zu einer Wuchshöhe von 3 bis 15 cm an und behandelt sie danach. Zur Nachauflaufbehandlung werden entweder direkt gesäte und in den gleichen Gefäßen aufgewachsenen Pflanzen ausgewählt, oder sie werden erst als Keimpflanzen getrennt angezogen und einige Tage vor der Behandlung in die Versuchsgefäße verpflanzt. Die Aufwandmengen für die Nachauflaufbehandlung variieren je nach Wirkstoff. Sie betragen 0,5, 2,0 oder 3,0 kg Wirkstoff/ha. Die Abdeckung unterbleibt bei der Nachauflaufbehandlung.

Die Versuchsgefäße werden im Gewächshaus aufgestellt, wobei für wärmeliebende Arten wärmere Bereiche (20 bis 35°C) und für solche gemäßigter Klimate 15 bis 25⁰C bevorzugt wurden. Die Versuchsperiode erstreckt sich über 2 bis 4 Wochen. Während dieser Zeit werden die Pflanzen gepflegt, und ihre Reaktion auf die einzelnen Behandlungen ausgewertet. Bewertet wird nach einer Skala von 0 bis 100. Dabei bedeutet 0 keine Schädigung oder normaler Ablauf und 100 kein Aufgang der Pflanzen bzw. völlige Zerstörung zumindest der oberirdischen Teile.

Die in den Versuchen verwendeten Pflanzen stammen von folgenden Arten: Amaranthus spp. (Fuchsschwanz), Avena sativa (Hafer), Cassia tora, Centaurea cyanus (Kornblume), Chenopodium album (Weißer Gänsefuß), Euphorbia geniculata (Südamerik. Wolfsmilchart), Galium aparine (Klettenlabkraut), Gossypium hirsutum (Baumwolle), Ipomoea spp. (Prunkwindearten), Sinapis alba (Weißer Senf), Solanum nigrum (Schwarzer Nachtschatten), Viola spp. (Stiefmütterchen), Viola tricolor (Gewöhnliches Stiefmütterchen).

Als Vergleichsmittel dienen die herbiziden Wirkstoffe 3-(4-Chlor-phenoxy)--1-diethylamino-n-propan (A; DE-OS 19 28 606) und 1-(4-Chlor-phenoxy)-1--diethylamino-n-butan (B; DE-OS 19 28 606) bzw. sie enthaltende herbizide Mittel.

Bei Vorauflaufanwendung zeigen beispielsweise die Verbindungen Nr. 21, 29 und 37 eine bessere herbizide Wirkung als die Vergleichsmittel. Selektivität in Hafer zeigen bei 3,0 kg Wirkstoff/ha die Verbindungen Nr. 2, 15, 18, 26, 28 und 31.

Bei Nachauflaufanwendung ist die herbizide Aktivität beispielsweise der Verbindungen Nr. 25, 29, 33 und 37 stärker als die der Vergleichsmittel. Die Verbindungen Nr. 25, 29 und 33 haben gleichzeitig eine gute Selektivität. Selektive herbizide Wirkung in Hafer zeigen bei 3,0 kg Wirkstoff/ha ferner die Verbindungen Nr. 7, 22, 77, 81, 84 und 87. Weiterhin sind die Verbindungen Nr. 18, 19, 38, 39 und 76 mit 3,0 kg Wirkstoff/ha bemerkenswert wirksam gegen breitblättrige unerwünschte Pflanzen.

In Anbetracht der guten Verträglichkeit der Wirkstoffe und der Vielseitigkeit der Applikationsmethoden können die erfindungsgemäßen Verbindungen oder diese enthaltende Mittel außer bei den in den Gewächshausversuchen getesteten Nutzpflanzen noch in einer weiteren großen Zahl von Kulturpflanzen zur Beseitigung unerwünschten Pflanzenwuchses eingesetzt werden.

In Betracht kommen beispielsweise die folgenden Kulturen:

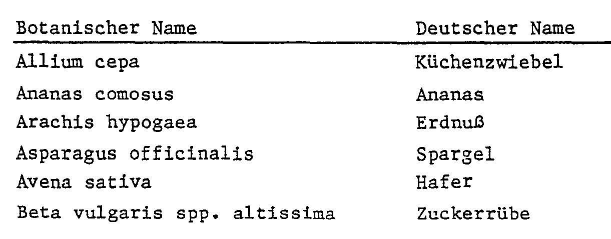

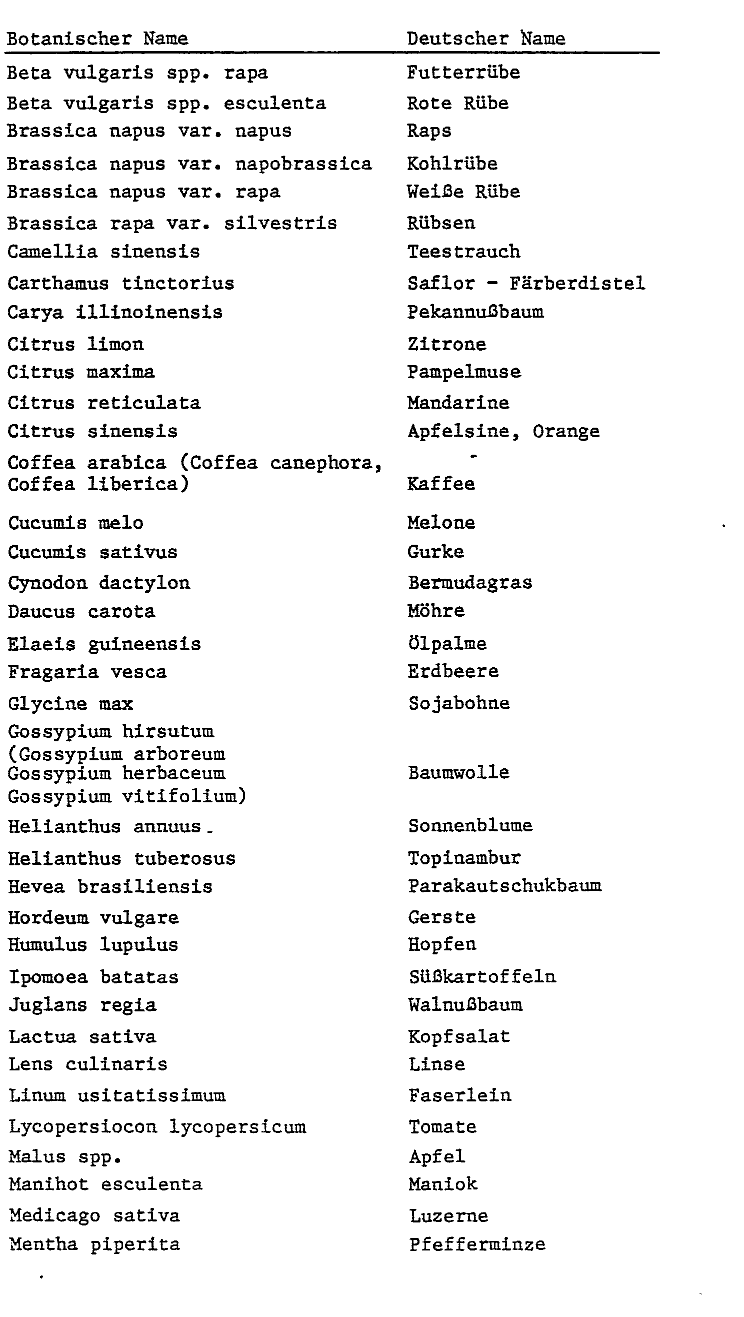

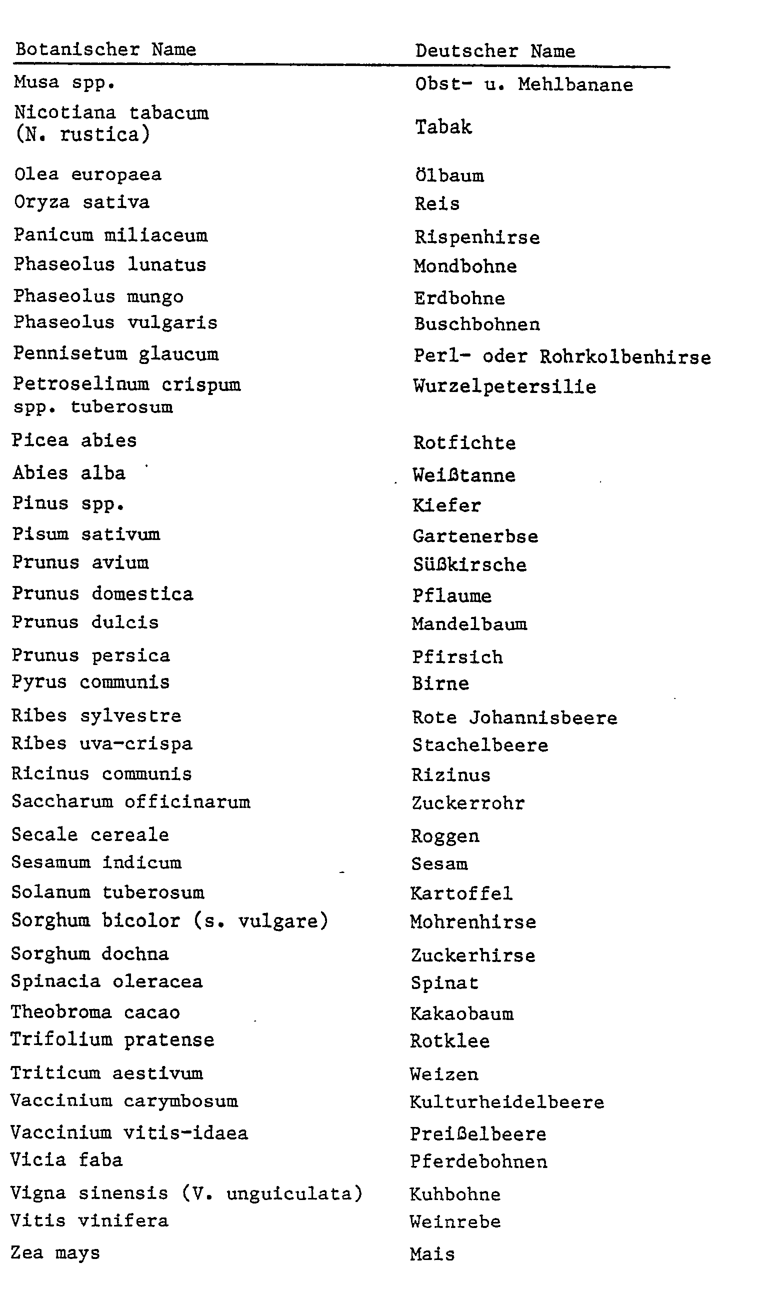

Zur Verbreiterung des Wirkungsspektrums und zur Erzielung synergistischer Effekte können die neuen erfindungsgemäßen Verbindungen mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate und andere in Betracht.

Außerdem ist es nützlich, die neuen Verbindungen allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- oder Spurenelementmängeln eingesetzt werden. Es können auch nichtphytotoxische öle und ölkonzentrate zugesetzt werden.

## Patentansprüche

1. Aryloxyalkylamine der Formel 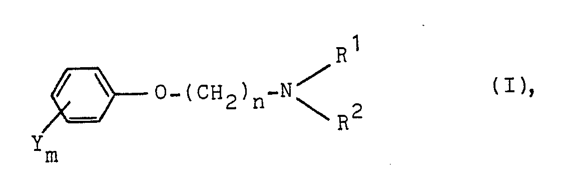 in der
Y Halogen, C_{I}-C₆-Alkyl, C₁-C₄-Halogenalkyl, C_{I}-C₄-Alkox^{y}, C₂-C₅--Alkanoyl, C₁-C₄-Alkylthio, C₁-C₄-Alkylsulfinyl, C₁-C₄-Alkylsulfonyl, Phenoxy, Benzyloxy oder Cyano,
m 1, 2 oder 3,
n eine ganze Zahl von 5 bis 12 und
R¹ und R² unabhängig voneinander Wasserstoff, gegebenenfalls durch C₁-C₄-Alkoxy, Hydroxy oder Cyano substituiertes C₁-C₄-Alkyl, C₂-C₄--Alkenyl, C₂-C₄-Alkinyl oder C₃-C₇-Cycloalkyl oder in der R¹ und R² zusammen eine gegebenenfalls durch C_{I}-C₄-Alkyl substituierte und/ oder durch Sauerstoff oder Schwefel unterbrochene Alkylenkette mit 4 bis 7 Kohlenstoffkettengliedern oder in der R¹ und R² zusammen mit dem Stickstoff, dessen Substituenten sie sind, einen gegebenenfalls durch Halogen oder C₁-C₄-Alkyl substituierten Pyridiniumrest, bedeuten
und in der anstelle des Restes 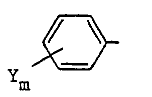
ein gegebenenfalls durch Ci-C₄-Alkyl, Formyl oder Hydroxy substituierter Naphthyl- oder 1,2,3,4-Tetrahydro-naphthylrest stehen kann,
mit der Maßgabe, daß n nicht 6 bedeutet, wenn Y t-Butyl und R¹ und R² zusammen Pentamethylen bedeuten,
und Salze dieser Aryloxyalkylamine.

2. Aryloxyalkylamine der Formel I gemäß Anspruch 1, dadurch gekennzeichnet, daß Y Halogen, m 1, n eine ganze Zahl von 6 bis 10 und R¹ und R² unabhängig voneinander C₁-C₄-Alkyl bedeuten, und Säureadditionszahle dieser Aryloxyalkylamine.

3. Salze der Aryloxyalkylamine der Formel I gemäß Anspruch 1, dadurch gekennzeichnet, daß Y Halogen oder C₁-C₄-Alkyl, m 2, n eine ganze Zahl von 6 bis 10 und R1 und R2 zusammen mit dem Stickstoff, dessen Substituenten sie sind, einen gegebenenfalls durch Halogen oder C₁-C₄-Alkyl substituierten Pyridiniumrest bedeuten.

4. Verfahren zur Herstellung der Aryloxyalkylamine der Formel I gemäß Anspruch 1, dadurch gekennzeichnet, daß man ein Phenoxyalkylhalogenid der Formel 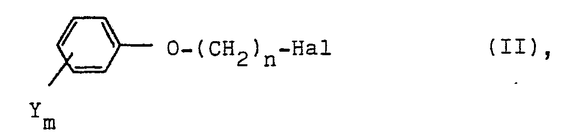 in der
Y, m und n die im Anspruch 1 genannten Bedeutungen haben und Hal für Halogen steht,
mit einem Amin der Formel 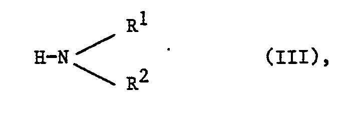 in der
R¹ und R² die im Anspruch'1 genannten Bedeutungen haben, gegebenenfalls in Gegenwart eines inerten Lösungsmittels umsetzt.

5. Verfahren zur Herstellung der Aryloxyalkylamine der Formel I gemäß Anspruch 1, dadurch gekennzeichnet, daß man ein Aminoalkylhalogenid der Formel 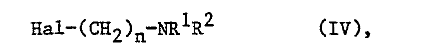 in der
R¹, R² und n die im Anspruch 1 genannten Bedeutungen haben und HaI für Halogen steht,
mit einem Alkalisalz eines Phenols der Formel 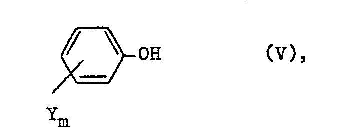 in der Y und m die im Anspruch 1 genannten Bedeutungen haben, in Gegenwart eines inerten Lösungsmittels umsetzt.

6. Herbizid, enthaltend ein Aryloxyalkylamin der Formel I oder ein Salz eines solchen Aryloxyalkylamins gemäß Anspruch 1.

7. Herbizid, enthaltend ein Aryloxyalkylamin der Formel I oder ein Säureadditionssalz eines solchen Aryloxyalkylamins gemäß Anspruch 2.

8. Herbizid, enthaltend ein Aryloxyalkylamin der Formel I oder ein Salz eines solchen Aryloxyalkylamins gemäß Anspruch 3.

9. Herbizid, enthaltend inerte Zusatzstoffe und ein Aryloxyalkylamin der Formel 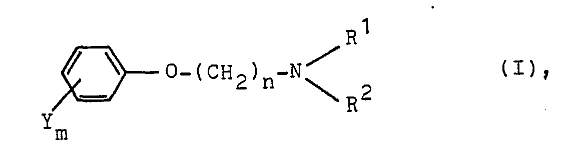 in der
Y Halogen, C₁-C₆-Alkyl, C₁-C₄-Halogenalkyl, C₁-C₄-Alkoxy, C₂-C₅--Alkanoyl, C₁-C₄-Alkylthio, C₁-C₄-Alkylsulfinyl, C_{I}-C₄-Alkylsulfonyl, Phenoxy, Benzyloxy oder Cyano,
m 1, 2 oder 3,
n eine ganze Zahl von 5 bis 12,
_{R}¹ und _{R}² unabhängig voneinander Wasserstoff, gegebenenfalls durch C₁-C₄-Alkoxy, Hydroxy oder Cyano substituiertes C₁-C₄-Alkyl, C₂-C₄--Alkenyl, C₂-C₄-Alkinyl oder C₃-C₇-Cycloalkyl oder in der R¹ und _{R}² zusammen eine gegebenenfalls durch C₁-C₄-Alkyl substituierte und/ oder durch Sauerstoff oder Schwefel unterbrochene Alkylenkette mit 4 bis 7 Kohlenstoffkettengliedern oder in der R¹ und R² zusammen mit dem Stickstoff, dessen-Substituenten sie sind, einen gegebenenfalls durch Halogen oder C₁-C₄-Alkyl substituierten Pyridiniumrest bedeuten
und in der anstelle des Restes 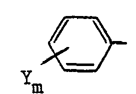
ein gegebenenfalls durch C_{I}-C₄-Alkyl, Formyl oder Hydroxy substituierter Naphthyl- oder 1,2,3,4-Tetrahydro-naphthylrest stehen kann,
oder ein Salz eines solchen Aryloxyalkylamins.

10. Verfahren zur Bekämpfung unerwünschten Pflanzenwuchses, dadurch gekennzeichnet, daß man eine herbizid wirksame Menge eines Aryloxyalkylamins der Formel I oder ein Salz eines solchen Aryloxyalkylamins gemäß Anspruch 1 auf die Pflanzen und/oder ihren Standort einwirken läßt.
